(19) Europäisches Patentamt
European Patent Office
Office européen des brevets

(11) Veröffentlichungsnummer: **0 346 767 A2**

(12) # EUROPÄISCHE PATENTANMELDUNG

(21) Anmeldenummer: **89110431.7**

(51) Int. Cl.4: **A61K 31/55**

(22) Anmeldetag: **09.06.89**

(30) Priorität: **15.06.88 DE 3820347**

(43) Veröffentlichungstag der Anmeldung: **20.12.89 Patentblatt 89/51**

(84) Benannte Vertragsstaaten: **AT BE CH DE FR GB IT LI LU NL SE**

(71) Anmelder: **Dr. Karl Thomae GmbH**
**Postfach 1755**
**D-7950 Biberach 1(DE)**

(72) Erfinder: **Mihm, Gerhard, Dr. Dipl.-Chem.**
**Nickeleshalde 5/1**
**D-7950 Biberach 1(DE)**
Erfinder: **Eberlein, Wolfgang, Dr. Dipl.-Chem.**
**Obere Au 6**
**D-7950 Biberach 1(DE)**
Erfinder: **Engel, Wolfhard, Dr. Dipl.-Chem.**
**Mozartstrasse 13**
**D-7950 Biberach 1(DE)**
Erfinder: **Trummlitz, Günter, Dr. Dipl.-Chem.**
**Buchenweg 27**
**D-7951 Warthausen(DE)**
Erfinder: **Mayer, Norbert, Dr.**
**Friedrich-Ebert-Strasse 66**
**D-7950 Biberach 1(DE)**
Erfinder: **Doods, Henri, Dr.**
**Hornsteinweg 7**
**D-7951 Warthausen(DE)**
Erfinder: **Hammer, Rudolf, Dr.**
**Grundstrasse 64**
**D-6507 Ingelheim/Rhein(DE)**

(54) **Verwendung von in 11-Stellung substituierten 5,11-Dihydro-6H-pyrido[2,3-b][1,4]benzodiazepin-6-onen zur Behandlung von Bradycardien und Bradyarrhythmien in der Human- und Veterinärmedizin.**

(57) In 11-Stellung substituierte 5,11-Dihydro-6H-pyrido[2,3-b][1,4]benzodiazepinone der allgemeinen Formel I

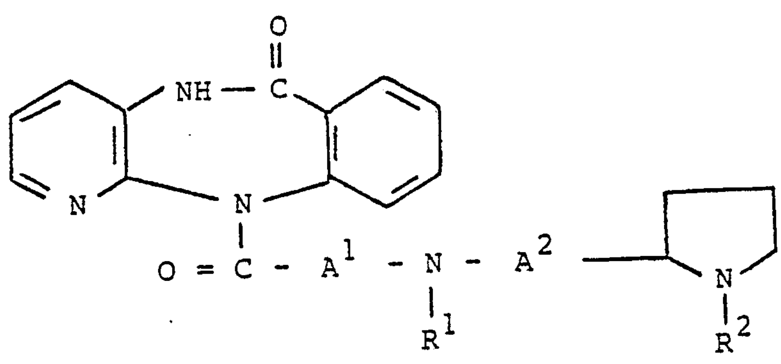

(I)

in der
$R^1$ und $R^2$ Wasserstoffatome oder Alkylgruppen mit 1 bis 3 Kohlenstoffatomen, $A^1$ eine geradkettige oder verzweigte Alkylengruppe mit 1 bis 5 Kohlenstoffatomen, $A^2$ eine Methylengruppe oder, wenn $R^1$ Wasserstoff und $R^2$ Methyl sind, auch eine Ethylengruppe sind, und ihre physiologisch verträglichen Säureadditionssalze eignen sich zur Behandlung von Bradycardien und Bradyarrhythmien und von Spasmen an Colon, Blase und Bronchien.

EP 0 346 767 A2

# Verwendung von in 11-Stellung substituierten 5,11-Dihydro-6H-pyrido[2,3-b][1,4]benzodiazepin-6-onen zur Behandlung von Bradycardien und Bradyarrhythmien in der Human- und Veterinärmedizin

Die Erfindung betrifft die Verwendung von 5,11-Dihydro-6H-pyrido[2,3-b][1,4]benzodiazepin-6-onen der allgemeinen Formel I

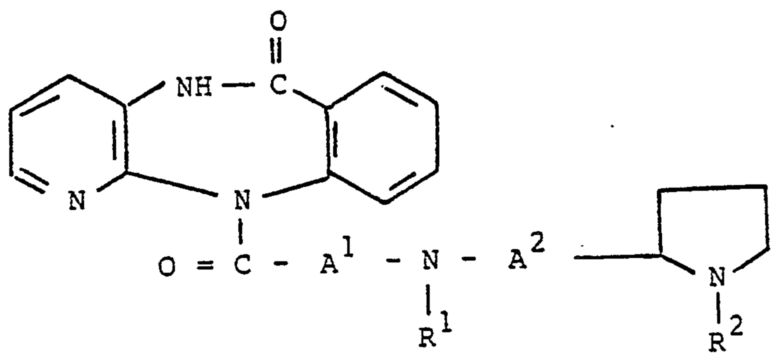

(I)

zur Behandlung von Bradycardien und Bradyarrhythmien in der Human- und Veterinärmedizin.

In der allgemeinen Formel I bedeuten

$R^1$ und $R^2$, die gleich oder voneinander verschieden sein können, Wasserstoffatome oder Alkylgruppen mit 1 bis 3 Kohlenstoffatomen,

$A^1$ eine geradkettige oder verzweigte Alkylengruppe mit 1 bis 5 Kohlenstoffatomen,

$A^2$ eine Methylengruppe oder, wenn $R^1$ Wasserstoff und $R^2$ Methyl sind, auch eine Ethylengruppe.

Einige der Verbindungen der obigen allgemeinen Formel I sind in der DE-C-2 724 478 (entspricht US-Patentschrift Nr. 4 210 648 oder GB-B-1 581 500) als ulkushemmende und magensaftsekretionshemmende Verbindungen beschrieben worden, des weiteren auch verschiedene Verfahren zu ihrer Herstellung. Die übrigen Verbindungen der allgemeinen Formel I lassen sich in völliger Analogie hierzu herstellen.

Es wurde nun überraschenderweise gefunden, daß die Verbindungen der allgemeinen Formel I und ihre Säureadditionssalze, insbesondere aber die Verbindungen

A = 5,11-Dihydro-11-[[[2-(1-methyl-2-pyrrolidinyl)ethyl]amino]acetyl]-6H-pyrido[2,3-b][1,4]benzodiazepin-6-on und

B = 5,11-Dihydro-11-[[[(1-ethyl-2-pyrrolidinyl)methyl]methylamino]acetyl]-6H-pyrido[2,3-b][1,4]-benzodiazepin-6-on und ihre Säureadditionssalze

günstige Effekte auf die Herzfrequenz ausüben und sich angesichts vergleichsweise geringer magensäuresekretionshemmender, salivationshemmender und mydriatischer Einflüsse als vagale Schrittmacher zur Behandlung von Bradycardien und Bradyarrhythmien in der Human- und Veterinärmedizin eignen. Einige Verbindungen zeigen auch spasmolytische Einflüsse auf periphere Organe, insbesondere Colon, Blase und Bronchien.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Bindungsstudien an muscarinischen Rezeptoren:

Bestimmung des $IC_{50}$-Wertes in vitro

Als Organspender dienten männliche Sprague-Dawley-Ratten mit 180-220 g Körpergewicht. Nach Entnahme von Herz und Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Großhirnrinde | 1:3000 |
| Submandibularis | 1: 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer betrug 45 Minuten. Als Radioligand wurde 0,3 nmolar $^{3}$H-N-Methylscopolamin ($^{3}$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^{3}$H-NMS. Der Anteil der unspezifischen Bindung wurde definiert als jene Radioaktivität, die in Anwesenheit von 1 $\mu$ molar Quinuclidinylbenzilat gebunden wurde. Es wurden immer Vierfachbestimmungen vorgenommen. Die $IC_{50}$-Werte der nicht-markierten Testsubstanzen wurden graphisch bestimmt. Sie repräsentieren jene Konzentration der Testsubstanz, bei welcher die spezifische Bindung von $^{3}$H-NMS an die muscarinischen Rezeptoren in den verschiedenen Organen um 50 % gehemmt wurde. Die Ergebnisse sind aus der Tabelle 1 zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. $M_1$-/$M_2$-Selektivität an der Ratte
2. Speichelsekretionshemmende Wirkung an der Ratte
3. Hemmung der Acetylcholinwirkung auf Blase, Bronchien und Herzfrequenz des Meerschweinchens

untersucht.

1. $M_1$-/$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle II.

2. Speichselsekretionshemmende Wirkung an der Ratte

Nach Lavy und Mulder (Arch. int. Pharmacodyn. 178, 437-445, (1969)) erhielten mit 1,2 g Urethan/kg narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Narkotisierten Meerschweinchen wurden 5 Minuten nach Gabe der Prüfsubstanz 10 µg/kg Acetylcholin sowohl intravenös als auch gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle III.

Nach den vorstehenden Angaben wurden beispielsweise die folgenden Verbindungen untersucht:

A = 5,11-Dihydro-11-[[[2-(1-methyl-2-pyrrolidinyl)ethyl]amino]acetyl]-6H-pyrido[2,3-b][1,4]benzodiazepin-6-on

B = 5,11-Dihydro-11-[[[(1-ethyl-2-pyrrolidinyl)methyl]methylamino]acetyl]-6H-pyrido[2,3-b][1,4]-benzodiazepin-6-on

und als Vergleichssubstanzen

C = 11-[[2-[(Diethylamino)methyl]-1-piperidinyl]acetyl]-5,11-dihydro-6H-pyrido[2,3-b][1,4]benzodiazepin-6-on (siehe US-Patent Nr. 4 550 107)

D = 5,11-Dihydro-11-[(4-methyl-1-piperazinyl)acetyl]-6H-pyrido[2,3-b][1,4]benzodiazepin-6-on (Pirenzepin, siehe US-Patent Nr. 3 660 380)

und

E = Atropin

Tabelle I:

| Rezeptor-Bindungs-Tests, in vitro: | | | |
|---|---|---|---|
| Ergebnisse: | | | |
| Substanz | Rezeptor-Bindungs-Tests $IC_{50}$[$nMl^{-1}$] | | |
| | Cortex | Herz | Submandibularis |
| A | 100 | 15 | 150 |
| B | 30 | 6 | 50 |
| C | 1200 | 140 | 5000 |
| D | 100 | 1500 | 200 |
| E | 2 | 4 | 4 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Großhirnrinde und Submandibularis.

Tabelle II:

| $M_1$-/$M_2$-Selektivität und Speichelsekretionshemmung an der Ratte: | | | |
|---|---|---|---|
| Ergebnisse: | | | |
| Substanz | -log $ED_{50}$[$Mkg^{-1}$] | | |
| | Herz | Blutdruck | Salivationshemmung |
| A | | | |
| B | 8,13 | 6,85 | 6,09 |
| C | 6,42 | 5,63 | 5,00 |
| D | 5,60 | 6,94 | 6,22 |
| E | 7,94 | 7,34 | 7,60 |

Tabelle III:

| Hemmung der Acetylcholinwirkung auf Blase, Bronchien und Herzfrequenz des Meerschweinchens: | | | |
|---|---|---|---|
| Ergebnisse: | | | |
| Substanz | -log $ED_{50}$[$Molkg^{-1}$] | | |
| | Herz | Bronchien | Blase |
| A | 7,01 | 6,99 | 6,27 |
| B | 7,34 | 7,46 | 5,93 |
| C | 5,84 | 5,58 | 4,73 |
| D | 5,85 | 6,57 | 5,36 |
| E | 7,70 | 7.96 | 7,03 |

Aus den pharmakologischen Daten der vorstehenden Tabellen II und III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird.

Außerdem deuten die pharmakologischen Daten der vorstehenden Tabelle III auf ein überraschend großes Unterscheidungsvermögen zwischen Herz und glatter Muskulatur.

Die genannten Substanzen weisen gegenüber der bereits bekannten Verbindung C eine wesentlich gesteigerte Wirkungsstärke auf. Dabei bleibt die therapeutisch nutzbare Selektivität erhalten. Dies führt zu einer geringeren Substanzbelastung des Patienten, ohne das Risiko muskarinischer Nebenwirkungen zu erhöhen.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Die Wirksubstanzen oder ihre physiologisch verträglichen Salze lassen sich in die üblichen pharmazeutischen Zubereitungsformen z. B. in Lösungen, Suppositorien, Tabletten, Dragées, Kapseln oder Teezubereitungen einarbeiten. Die Einzeldosis liegt im allgemeinen zwischen 0,002 und 5 mmg/kg, vorzugsweise 0,05 und 1,0 mg/kg Körpergewicht, die gegebenenfalls auch in Form mehrerer auch zeitlich versetzter Einzelgaben zur Erzielung des gewünschten therapeutischen Effekts verabreicht wird.

Die Wirksubstanzen können mit Hilfe entsprechender anorganischer oder organischer Säuren auch in ihre physiologisch verträglichen Salze übergeführt werden. Als Säuren haben sich beispielsweise Salzsäure, Bromwasserstoffsäure, Schwefelsäure, Phorsphorsäure, Weinsäure. Fumarsäure, Zitronensäure, Maleinsäure, Bernsteinsäure, Äpfelsäure als geeignet erwiesen.

Die folgenden Beispiele sollen die Herstellung einiger pharmazeutischer Anwendungsformen verdeutlichen:

Beispiel I

Tabletten mit 50 mg 5,11-Dihydro-11-[[[2-(1-methyl-2-pyrrolidinyl)ethyl]amino]acetyl]-6H-pyrido[2,3-b][1,4]-benzodiazepin-6-on

| Zusammensetzung: | |
|---|---|
| 1 Tablette enthält: | |
| Wirkstoff | 50,0 mg |
| Milchzucker | 148,0 mg |
| Kartoffelstärke | 65,0 mg |
| Magnesiumstearat | 2,0 mg |
| | 265,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 50 mg 5,11-Dihydro-11-[[[2-(1-methyl-2-pyrrolidinyl)ethyl]amino]acetyl]-6H-pyrido[2,3-b][1,4]-benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 5,11-Dihydro-11-[[[2-(1-methyl-2-pyrrolidinyl)ethyl]amino]acetyl]-6H-pyrido[2,3-b][1,4]-benzodiazepin-6-on-dihydrochlorid

| Zusammensetzung: | |
|---|---|
| 1 Ampulle enthält: | |
| Wirkstoff | 10,0 mg |
| Natriumchlorid | 8,0 mg |
| Dest. Wasser | ad 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120°C.

Beispiel IV

Suppositorien mit 50 mg 5,11-Dihydro-11-[[[(1-ethyl-2-pyrrolidinyl)methyl]methylamino]acetyl]-6H-pyrido-[2,3-b][1,4]-benzodiazepin-6-on

| Zusammensetzung: | |
|---|---|
| 1 Zäpfchen enthält: | |
| Wirkstoff | 50,0 mg |
| Zäpfchenmasse (z.B. Witepsol W 45(R)) | 1 695,0 mg |
| | 1 745,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.
Zäpfchengewicht 1,745 g

Beispiel V

Tropfen mit 5,11-Dihydro-11-[[[(1-ethyl-2-pyrrolidinyl)methyl]methylamino]acetyl]-6H-pyrido[2,3-b][1,4]-benzodiazepin-6-on-dihydrochlorid

| Zusammensetzung: | |
|---|---|
| 100 ml Tropflösung enthalten: | |
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 5,0 g |
| Natriumcyclamat | 1,0 g |
| Glycerin | 15,0 g |
| Dest. Wasser | ad 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Ansprüche

1.) Verwendung von in 11-Stellung substituierten 5,11-Dihydro-6H-pyrido[2,3-b][1,4]benzodiazepin-6-onen der allgemeinen Formel I

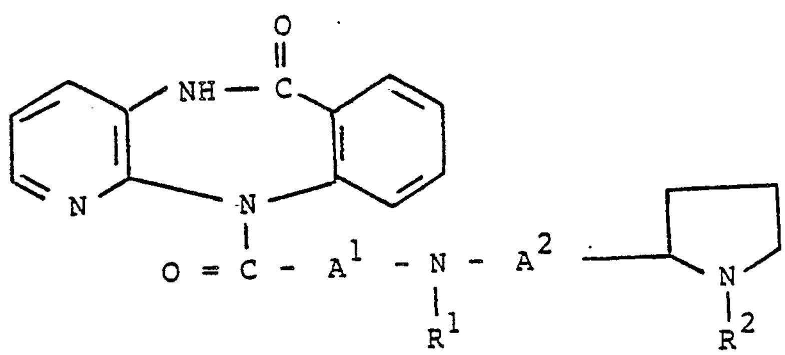

(I)

in der

$R^1$ und $R^2$, die gleich oder voneinander verschieden sind, Wasserstoffatome oder Alkylgruppen mit 1 bis 3 Kohlenstoffatomen und $A^1$ eine geradkettige oder verzweigte Alkylengruppe mit 1 bis 5 Kohlenstoffatomen, $A^2$ eine Methylengruppe oder, wenn $R^1$ Wasserstoff und $R^2$ Methyl sind, auch eine Ethylengruppe bedeuten, und ihren physiologisch verträglichen Säureadditionssalzen zur Behandlung von Bradycardien und Bradyarrhythmien und von Spasmen an Colon, Blase und Bronchien in der Human- und Veterinärmedizin.

2.) Verwendung von in 11-Stellung substituierten 5,11-Dihydro-6H-pyrido[2,3-b][1,4]benzodiazepin-6-onen der allgemeinen Formel I

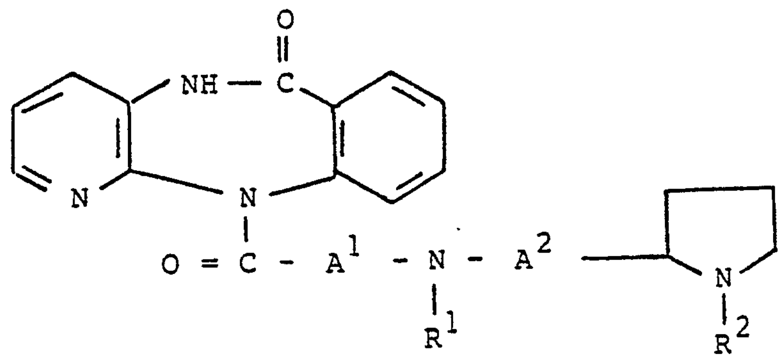

(I)

in der

$R^1$ und $R^2$, die gleich oder voneinander verschieden sind, Wasserstoffatome oder Alkylgruppen mit 1 bis 3 Kohlenstoffatomen und $A^1$ eine geradkettige oder verzweigte Alkylengruppe mit 1 bis 5 Kohlenstoffatomen, $A^2$ eine Methylengruppe oder, wenn $R^1$ Wasserstoff und $R^2$ Methyl sind, auch eine Ethylengruppe bedeuten, und ihren physiologisch verträglichen Säureadditionssalzen zur Herstellung eines Arzneimittels für die Behandlung von Bradycardien und Bradyarrhythmien und von Spasmen an Colon, Blase und Bronchien in der Human- und Veterinärmedizin.

3.) Verwendung von 5,11-Dihydro-11-[[[2-(1-methyl-2-pyrrolidinyl)ethyl]amino]acetyl]-6H-pyrido[2,3-b]-[1,4]benzodiazepin-6-on und seinen physiologisch verträglichen Säureadditionssalzen zur Behandlung von Bradycardien und Bradyarrhythmien und von Spasmen an Colon, Blase und Bronchien.

4.) Verwendung von 5,11-Dihydro-11-[[[(1-ethyl-2-pyrrolidinyl)methyl]methylamino]acetyl]-6H-pyrido[2,3-b][1,4]benzodiazepin-6-on und seinen physiologisch verträglichen Säureadditionssalzen zur Behandlung von Bradycardien und Bradyarrhythmien und von Spasmen an Colon, Blase und Bronchien.

5.) Verwendung von 5,11-Dihydro-11-[[[2-(1-methyl-2-pyrrolidinyl)ethyl]amino]acetyl]-6H-pyrido[2,3-b]-[1,4]benzodiazepin-6-on und seinen physiologisch verträglichen Säureadditionssalzen zur Herstellung eines Arzneimittels für die Behandlung von Bradycardien und Bradyarrhythmien und von Spasmen an Colon, Blase und Bronchien.

6.) Verwendung von 5,11-Dihydro-11-[[[(1-ethyl-2-pyrrolidinyl)methyl]methylamino]acetyl]-6H-pyrido[2,3-b][1,4]benzodiazepin-6-on und seinen physiologisch verträglichen Säureadditionssalzen zur Herstellung eines Arzneimittels für die Behandlung von Bradycardien und Bradyarrhythmien und von Spasmen an Colon, Blase und Bronchien.